# EUROPEAN PATENT APPLICATION

(11) **EP 2 724 704 A1**
(43) Date of publication of application: **30.04.2014**
(21) Application number: 13193062.0
(22) Date of filing: 21.12.2006
(51) Int. Cl.: A61J 3/06, A61K 9/14, A61K 31/4709, A61K 31/522, A61K 47/12, A61K 47/14, A61K 47/44, B01J 2/04, B01J 2/20

(54) **Method of producing drug-containing wax matrix particles, extruder to be used in the method and sustained-release preparation containing cilostazol**

(30) Priority: 22.12.2005 JP 2005370927; 05.06.2006 JP 2006156578
(62) Divisional of application: 06835067.7
(71) Applicant: OTSUKA PHARMACEUTICAL CO., LTD., Chiyoda-ku Tokyo 101-8535 (JP)
(72) Inventor: Tomohira, Yuso, Tokushima, 771-0182 (JP); Yamaguchi, Yasuo, Tokushima, 771-0182 (JP)
(74) Representative: HOFFMANN EITLE

(57) **Abstract**

A sustained-release preparation is provided which contains granules comprising (A) cilostazol crystals; and (B) glycerol fatty acid ester and/or polyglycerol fatty acid ester. The average particle diameter of the granules ranges from 40 to 200 µm.

## Description

### TECHNICAL FIELD

The present invention relates to a method for producing drug-containing wax matrix granules. The invention also relates to an extruder for producing drug-containing wax matrix granules.

The present invention also relates to cilostazol-containing sustained-release preparations. More specifically, the present invention relates to preparations comprising wax matrix granules containing cilostazol, and relates to sustained-release preparations having an outstanding sustained-release property and in which differences in cilostazol release and blood concentration change occurring between administration while fasting and administration after food intake are small.

### BACKGROUND ART

As oral sustained-release preparations, tablet-like single-unit preparations and granular multiple-unit preparations are known. In view of the drug release in the body and the blood concentration profile, multiple-unit preparations have small variations between individuals, and thus are preferable. Suitable as agents for imparting a sustained-release property to a drug are hydrophilic hydrogel preparations using water soluble polymers, such as hydroxypropylmethylcellulose (HPMC), hydroxypropylcellulose (HPC), and polyethylene oxide (PEO). In such hydrophilic hydrogel preparations, however, drug release is liable to be affected by food intake. Film coating by a pH dependent or non-dependent polymer is suitable for both tablets and granules in view of adjusting the solubility of a preparation. In such film coating, however, the influence of variations in endogastric acidity is not negligible, and there are problems with production abilities (i.e., coating time, yield, variation between lots) for granules having a small particle diameter, in particular fine particles whose particle diameter is 100 µm or less. In addition, film coating preparations comprising water-insoluble polymers, such as ethyl cellulose (EC), are difficult to apply to the film coating of fine particles and are also not suitable for imparting a sustained-release property to low-solubility drugs.

It is also known that a sustained-release property can be imparted to a drug using a wax(s), which is water-insoluble oil or fat-based substances, as a matrix base material. Granules containing such a matrix base material (hereafter sometimes referred to as wax matrix granules) are known to be useful as sustained-release preparations.

Hitherto-known wax matrix-based preparations include wax matrix granules having a diameter of 1 mm or less and wax matrix pellets or tablets having a diameter of several millimeters. Such wax matrix-based preparations are produced by a melting method, spraying method, heat-melt-spraying method, kneading extrusion method, etc. Among these hitherto-known methods, a heat-melt-spraying method is known as a method for producing wax matrix granules having an average particle diameter of 1 mm or less. Specifically, the method has the steps of heating a substance having a low melting point at temperatures higher than its melting point to melt the substance, adding a drug and another additive to the molten substance, and spray cooling the resulting mixture using a large-sized spray air cooler (see Patent Document 1).

This heat-melt-spraying method is suitable for forming spherical matrix granules. The method, however, has the following disadvantages: (1) a large-sized spray cooling device is required; (2) a heating and melting tank needs to have a device for uniformly mixing starting materials therein; (3) temperature control is required for a pump and the piping that connects the tank to the spray cooling device; etc. In the case of mass production according to the heat-melt-spraying method, since a large amount of starting materials need to be heated, the starting materials are inevitably heated at high temperatures for a prolonged period time. Thus, the stability of the drug or additive may be degraded by heat. The heat-melt-spraying method also has another disadvantage when a drug dissolved or melted in a molten mixture is likely to precipitate out. Specifically, troubles caused by solidification of the precipitated drug arise on the liquid contacting surface between the molten mixture liquid and the tank surface or the tank wall surface; a liquid supply line; a spray unit of a rotation disk, a spray nozzle, and the like; etc.

Patent Document 2 discloses a method for producing wax matrix granules using a multi-screw extruder. The method of Patent Document 2 provides wax matrix granules having an average particle diameter of 1 mm or less. The method has the steps of controlling the temperature of a substance discharged from a die so it is lower than the melting point of the wax (preferably, a temperature that is lower than the melting point by 10 to 20°C) ; cutting the discharged substance into pellets with a high-speed cutter while solidifying it by cooling; and then pulverizing the pellets with a roll granulator; and the like. This method makes it possible to transform starting materials into a matrix shape in a single step by the use of a multi-screw extruder. However, there are disadvantages in that, in order to obtain granules having a diameter of 1 mm or less, a pulverization process is separately required and the pulverization process cannot produce spherical granules.

In addition, the following methods are known as methods for producing a wax matrix preparation using an extruder: a method having the step of cutting a substance discharged from an extruder with a high-speed cutter in such a manner as to have a round shape (e.g., Patent Document 3); a method for forming a substance discharged from an extruder into a lenticular tablet (e.g., Patent Document 4); and a method having the steps of pulverizing a substance discharged from an extruder with a mill, and further cutting the pulverized substance with a high-speed cutter while cooling it using water and/or air (e.g., Non-Patent Document 1). However, these methods using extruders have other disadvantages in that there is variation in granule forms and therefore spherical granules cannot be obtained, in addition to the above-described disadvantage of the method of Patent Document 1, i.e., separately requiring a pulverization process in order to obtain wax matrix granules having an average particle diameter of 1 mm or less. Moreover, Patent Document 5 discloses a method having the steps of melting and mixing starting materials with a twin-screw extruder, feeding the molten mixture to an atomizer with a pump, and spraying the molten mixture. This method requires feeding a liquid with a pump, which causes troubles such as liquid blockage in the pipe connecting portion due to the crystallization of the molten drug, and the like. Therefore, this method has disadvantages such as the increased burden of maintaining a device, reduced production efficiency, and the like.

In view of the above-described prior art techniques, the need exists for establishing a method to produce, by a simple method, drug-containing wax matrix granules, particularly drug-containing wax matrix granules having an average particle diameter of 1 mm or less, while avoiding liquid blockage due to the recrystallization of a dissolved or melted drug during the period from a melting step to a spraying step.

Previously known drug-containing wax matrix granules have both the above-described production problems and disadvantages in that the containable drugs are limited, resulting in limited clinical applications. More specifically, containable drugs are limited to those with relatively high water solubility of about 0.6 w/v%, such as theophylline because it is important for the drug contained in the wax matrix granules to be completely released and absorbed in the body for the limited period of time during which the granules travel through the gastrointestinal tract. In general, it is known that preparations comprising wax matrix granules have a tendency to increase the differences in drug release and blood concentration change between administration while fasting and administration after food intake since the breakdown rate of the wax matrix base material increases due to the digestive process in the gastrointestinal tract. Although such a disadvantage can be overcome by strictly setting the administration time of the preparation, the observance of the administration time of the preparation is left to the self-control of each patient. Thus, the above-described disadvantages need to be overcome in view of properties of the preparation as well.

In contrast, cilostazol is a low-solubility drug used as an antiplatelet agent with a peripheral vasodilator action for treating ulcer based on chronic occlusive diseases and ameliorating ischemia symptoms, such as sharp pains, sensitivity to cold, etc. Heretofore, a method for producing a preparation containing cilostazol as a sustained release preparation has been proposed in Patent Document 6, but no method for producing a preparation using wax matrix granules containing cilostazol as a sustained release preparation has been reported. In order to effectively exhibit the desired drug effects of cilostazol-containing sustained-release preparations, it is important that cilostazol be released in the lower part of the gastrointestinal tract. To this end, cilostazol having a particle diameter of several micrometers needs to be contained in a sustained release preparation.

Thus, there is also a need to develop pharmaceutical preparations comprising wax matrix granules containing cilostazol; in which differences in cilostazol release and blood concentration change occurring between administration while fasting and administration after food intake are small; and that have an excellent sustained release.
[Patent Document 1] Japanese patent No. 2973751
[Patent Document 2] Japanese patent No. 2616252
[Patent Document 3] Japanese Unexamined Patent Publication No. 1998-57450
[Patent Document 4] Japanese translation of PCT international application No. 1998-511289
[Patent Document 5] Japanese Unexamined Patent Publication No. 2005-162733
[Patent Document 6] Japanese Unexamined Patent Publication No. 2001-163769
[Non-Patent Document 1] Pharmaceutical Extrusion Technology, edited by Isaac Ghebre-Sellassie, Charles Martin, DRUGS AND THE PHARMACEUTICAL SCIENCE VOL.133, MARCEL DEKKER, INC, 2003, and Chapter 9, pages 171-181

### DISCLOSURE OF THE INVENTION

### PROBLEMS TO BE SOLVED BY THE INVENTION

The invention aims to provide a method for producing, by a simple method, drug-containing wax matrix granules, particularly drug-containing wax matrix granules having an average particle diameter of 1 mm or less, while avoiding liquid blockage due to the recrystallization of a molten drug during the period from a melting step to a spraying step. The invention also aims to provide a device capable of easily producing drug-containing wax matrix granules. In addition, the present invention aims to provide preparations comprising cilostazol-containing wax matrix granules that have an excellent sustained release and in which differences in cilostazol release and blood concentration change occurring between administration while fasting and administration after food intake are small.

### MEANS FOR SOLVING THE PROBLEMS

The present inventors conducted extensive research to overcome the above-described problems, and found that drug-containing wax matrix granules can be produced using an extruder onto which a spray nozzle capable of spraying and discharging starting materials is directly mounted. More specifically, the inventors found that drug-containing wax matrix granules can be produced by supplying at least one drug and at least one wax to an extruder; adjusting the temperature of a barrel and a die to be higher than the melting point of the wax; spraying and discharging the drug and the wax into the air from a spray nozzle mounted on a die provided at a top end of the barrel of the extruder while melting and kneading; and solidifying the molten and kneaded drug and wax by cooling them in the air. The present inventors made further improvements based on these findings, and accomplished the present invention. The present inventors also found that preparations comprising wax matrix granules which have (A) cilostazol crystals and (B) glycerol fatty acid ester and/or polyglycerol fatty acid ester and whose average particle diameter ranges from 40 to 200 µm are excellent in the release of cilostazol, and, in such preparations, differences in cilostazol release and blood concentration change occurring between administration while fasting and administration after food intake are small.

More specifically, the present invention includes the following aspects.
Item 1. A method for producing drug-containing wax matrix granules comprising at least one drug and at least one wax, the method comprising the steps of:
   (i) supplying the at least one drug and the at least one wax to an extruder in which the temperature of a barrel and the temperature of a die are adjusted to be higher than the melting point of the at least one wax; and
   (ii) while melting and kneading the at least one drug and the at least one wax in the extruder to give a molten kneaded mixture of the drug and wax, spraying the molten kneaded mixture of the drug and wax into an atmosphere having a temperature lower than the melting point of the wax from a spray nozzle directly mounted onto a die provided at a top end of the barrel of the extruder, thereby forming the mixture into granules.
Item 2. The method according to item 1, wherein the spray nozzle is a single-fluid nozzle, pressurization nozzle, two-fluid nozzle, or multi-fluid nozzle.
Item 3. The method according to item 1, wherein the extruder is a single-screw extruder, twin-screw extruder, or multi-screw extruder having at least three screws.
Item 4. The method according to item 1, wherein the drug-containing wax matrix granules are spherical.
Item 5. The method according to item 4, wherein the drug-containing wax matrix granules have an average particle diameter of 1 mm or less.
Item 6. The method according to item 1, wherein the at least one drug is at least one member selected from the group consisting of theophylline, cilostazol, ketoprofen, naproxen, diclofenac, itraconazole, piroxicam, phenytoin, verapamil, probucol and tolvaptan.
Item 7. The method according to item 1, wherein the at least one wax is at least one member selected from the group consisting of paraffin, micro crystallin wax, ceresin, Japan wax, cacao butter, carnauba wax, beeswax, cetanol, steryl alcohol, myristic acid, palmitic acid, stearic acid, glycerine fatty acid ester, polyglycerin fatty acid ester, glycerin organic-acid fatty acid ester, propylene glycol fatty acid ester, sorbitan fatty acid ester and hydrogenated oil.
Item 8. The method according to item 1, wherein
   the drug-containing wax matrix granules have 0.001 to 90% by weight of drug and 0.1 to 99.99% by weight of wax based on a total amount of the granules.
Item 9. The method according to item 1, wherein
   (A) the at least one drug is cilostazol;
   (B) the at least one wax is glycerol fatty acid ester and/or polyglycerol fatty acid ester; and
   an average particle diameter of the drug-containing wax matrix granules ranges from 40 to 200 µm.
Item 10. The method according to item 9, further comprising the step (iii) of heating the granules prepared in the step (ii) at a temperature of 40 to 55°C.
Item 11. The production method according to item 10, wherein, in the step (iii), inert particles are adhered to a surface of the granules obtained in the step (ii) before heating the granules at a temperature of 40 to 55°C.
Item 12. An extruder for producing drug-containing wax matrix granules, the extruder comprising:
   a barrel having a temperature controller;
   a supply port for supplying at least one drug and at least one wax to the barrel;
   an outlet die provided in the barrel;
   an extrusion screw for preparing a molten kneaded mixture of the at least one drug and the at least one wax and conveying the mixture to the outlet die, the extrusion screw being disposed within the barrel; and
   a spray nozzle capable of spraying the molten kneaded mixture of the drug and wax, the spray nozzle being directly mounted on the outlet die.
Item 13. The extruder for producing drug-containing wax matrix granules according to item 12 further having a granule-forming chamber for solidifying the molten kneaded mixture of the drug and wax discharged from the spray nozzle to form granules.
Item 14. A sustained-release preparation containing granules comprising:
   (A) cilostazol crystals; and
   (B) glycerol fatty acid ester and/or polyglycerol fatty acid ester; and
      an average particle diameter of the granules ranges from 40 to 200 µm.
Item 15. A sustained-release preparation according to item 14, wherein an average particle diameter of the (A) cilostazol crystals is 10 µm or less.
Item 16. A sustained-release preparation according to item 14, wherein (A) the cilostazol crystals are present in a proportion of 5 to 60% by weight and (B) the glycerol fatty acid ester and/or polyglycerol fatty acid ester is/are present in a proportion of 30 to 95% by weight based on a total amount of the granules in the sustained-release preparation.
Item 17. A sustained-release preparation according to item 14, further comprising a water-soluble cellulose derivative. Item 18. A sustained-release preparation according to item 17, wherein the water-soluble cellulose derivative is hydroxypropylmethylcellulose.
Item 19. A sustained-release preparation according to item 17, comprising 1 to 15% by weight of the water-soluble cellulose derivative based on a total amount thereof.
Item 20. A sustained-release preparation according to item 18, comprising 1 to 15% by weight of hydroxypropylmethylcellulose based on a total amount thereof.
Item 21. A sustained-release preparation according to item 14, wherein the granules comprising the ingredients (A) and (B) are granules prepared by solidifying a molten mixture of the ingredients (A) and (B).
Item 22. A sustained-release preparation according to item 14, wherein inert particles are adhered to a surface of the granules. Item 23. A sustained-release preparation containing cilostazol according to item 22, wherein the inert particle is at least one member selected from the group consisting of talc, light anhydrous silicic acid, titanium oxides, and cellulose-based polymers. Item 24. A sustained-release preparation according to item 14, wherein the ingredient (B) is at least one member selected from the group consisting of glycerol stearate, polyglycerol stearate, glycerol behenate, and polyglycerol behenate.
Item 25. A sustained-release preparation according to item 14, wherein the ingredient (B) is at least one member selected from the group consisting of glycerol behenate, diglycerol stearate, and triglyceryl half-ester of behenic acid.
Item 26. A sustained-release preparation according to item 14 prepared by steps (i) and (ii);
   (i) supplying (A) cilostazol and (B) glycerol fatty acid ester and/or polyglycerol fatty acid ester to an extruder in which the temperature of a barrel and the temperature of a die are adjusted to be higher than the melting point of the ingredient (B); and
   (ii) while melting and kneading the ingredients (A) and (B) in the extruder to give a molten kneaded mixture of the ingredients (A) and (B), spraying the molten kneaded mixture of the ingredients (A) and (B) into an atmosphere having a temperature lower than the melting point of the ingredient (B) from a spray nozzle directly mounted onto a die provided at a top end of the barrel of the extruder, thereby forming the mixture into granules. Item 27. A sustained-release preparation according to item 26 prepared by supplying (C) a water-soluble cellulose derivative in addition to the ingredients (A) and (B) in the step (i).
Item 28. A sustained-release preparation according to item 26, prepared by further subjecting the granules obtained in the step (ii) to the step (iii) of heating the granules at 40 to 55°C. Item 29. A sustained-release preparation according to item 26 prepared by adhering, prior to the heating step in the step (iii), inert particles to a surface of the granules obtained in the step (ii).

### EFFECTS OF THE INVENTION

According to the production method and extruder of the invention, it is possible to produce drug-containing wax matrix granules, particularly drug-containing wax matrix granules having an average particle diameter of 1 mm or less, while avoiding blockage of a liquid supply line (piping) due to recrystallization of a dissolved or molten drug. Moreover, according to the production method and extruder of the invention, drug-containing wax matrix granules can be easily produced in a single step without undergoing another step such as a pulverization process or the like, and thus are also highly useful in the respect of the industrial application.

It is indispensable for the granules (i.e. wax matrix granules) contained in the sustained-release preparation of the invention to (1) comprise cilostazol crystals (whose average particle diameter is preferably 10 µm or less), (2) containing glycerol fatty acid ester and/or polyglycerol fatty acid ester as a wax matrix base material, and (3) have an average particle diameter of 40 to 200 µm for the wax matrix granules. The sustained-release preparation of the invention thus prepared can impart an excellent sustained-release property to cilostazol, which is a low solubility drug, and can reduce differences in the release of cilostazol and blood concentration changes between administration while fasting and administration after food intake. Accordingly, the sustained-release preparation of the invention can more effectively exhibit the drug effects of cilostazol, and useful as a pharmaceutical preparation.

Moreover, the sustained-release preparation of the invention can be imparted with high bioavailability while maintaining a sustained-release property by adding a water-soluble cellulose derivative, particularly hydroxypropylmethylcellulose, and therefore, has extremely high clinical efficacy.

### BEST MODE FOR CARRYING OUT THE INVENTION

In the invention, a drug-containing wax matrix refers to a composition in which a drug is embedded in a continuous phase wax while being dissolved or dispersed, and "sustained-release preparation" refers to a preparation showing sustained release property of the drug contained therein when orally administered. Hereinafter, the invention will be described in detail.

### 1. Method for producing drug containing wax-matrix granules Extruder

The production method of the invention is conducted using an extruder having a spray nozzle mounted on an outlet die provided at the top end of a barrel.

Various aspects of an extruder preferably used in the production method of the invention (i.e., an extruder for producing drug-containing wax matrix granules) will be described.

The extruder has a barrel (cylinder) with a temperature control member. There is no limitation on the temperature control member insofar as the temperature of a wax to be supplied into the barrel is raised above its melting point. It is preferable that the temperature control member further have a cooling function. With the cooling function, the temperature inside the barrel can be controlled more appropriately. A barrel jacket capable of heating and/or cooling can be mentioned as a specific example of such temperature control means.

In the barrel of the extruder, a supply port for supplying starting materials is provided at the upstream side and an outlet die for discharging a molten kneaded mixture of the starting materials is provided at the downstream side.

The barrel of the extruder has, in its interior, an extrusion screw for preparing a molten kneaded mixture of the starting materials supplied from the supply port and conveying the molten kneaded mixture of the starting materials to the outlet die. There is no limitation on the number of extrusion screws, and either an extrusion screw of a single-screw type, a twin-screw type, or a multi-screw type of three or more screws may be used, preferably a twin-screw type.

The form of the screws is not limited insofar as the starting materials supplied from the supply port can be formed into a molten kneaded mixture and further the molten kneaded mixture of the starting materials can be conveyed. For example, conveyor screws, kneading screws, mixing screws, or the combined use thereof can be mentioned.

The outlet die of the extruder has a spray nozzle in such a manner that the molten kneaded mixture of the starting materials that have been conveyed to the outlet die with a screw is sprayed and discharged to the outside.

The spray nozzle is not limited in its spraying manner, and a pressurization nozzle, two-fluid nozzle, or a multi-fluid nozzle having at least two fluids may be used. There is no limitation on the form of the discharge port of the spray nozzle insofar as the molten kneaded mixture of the starting materials can be sprayed and discharged, and a round shape can be mentioned as a preferable example. When the discharge port has a round shape, the inner diameter is, for example, 0.1 to 20 mm, preferably 0.2 to 15 mm, and more preferably 0.2 to 10 mm.

It is preferable that the extruder have a heating member for heating the spray-air (the air used for spraying molten kneaded mixture of the starting materials) to be supplied to the spray nozzle.

Moreover, it is preferable that the extruder further have a granule-forming chamber in which the molten kneaded mixture of the starting materials sprayed from the spray nozzle is solidified and formed into granules. There is no limitation on the manner of providing the granule-forming chamber to the extruder, insofar as the molten kneaded mixture of the starting materials can be discharged in the granule-forming chamber from the discharge port of the spray nozzle. For example, the granule-forming chamber can be provided in such a manner that the discharge port of the spray nozzle is inserted into and attached to the granule-forming chamber. There is no limitation on the form of the granule-forming chamber insofar as the molten kneaded mixture of the starting materials is solidified and formed into granules in the chamber. For example, the granule-forming chamber may be configured so that the molten kneaded mixture of the starting materials is sprayed from the spray nozzle in an atmosphere in the chamber. The temperature inside the chamber may be controlled by a liquid, such as liquid nitrogen, that is held within the chamber. The granule-forming chamber is also equipped with a wax matrix granule collector for collecting wax matrix granules formed within the chamber. The granule-forming chamber preferably has a temperature control member for controlling the atmospheric temperature of the molten kneaded mixture of the starting materials to be sprayed in the chamber, and preferably has an air-exhaust member for exhausting the spray air introduced into the chamber. The air-exhaust member may have a wax matrix granule collector for collecting any wax matrix granules remaining in the exhausted air.

Hereafter, preferable examples of the extruder will be described with reference to drawings.

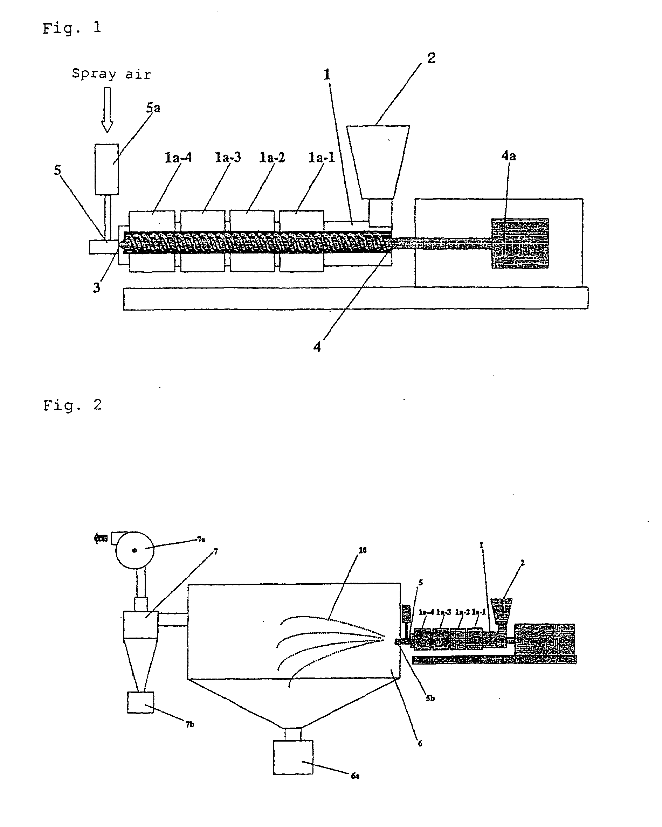

The extruder shown in Fig. 1 has a barrel 1 equipped with four barrel jackets 1a capable of controlling the temperature as a temperature control member. The barrel jackets are designated by the reference numerals of 1a-1, 1a-2, 1a-3, and 1a-4, as viewed from the upstream side. The barrel 1 has a supply port 2 for supplying starting materials to the upstream side and has an outlet die 3 at the downstream side. The barrel 1 has a screw 4 for conveying starting materials supplied from a supply port 2 to the outlet die 3 while the starting materials are melted and kneaded. The screw 4 is configured to be driven by a motor 4a. The outlet die 3 is equipped with a spray nozzle 5 having a heating member 5a for heating spray air and a discharge port 5b for discharging the molten kneaded mixture of the starting materials.

Fig. 2 shows an example of an extruder having a granule-forming chamber 6 configured so that the molten kneaded mixture of the starting materials is sprayed in a gas atmosphere from the spray nozzle. In Fig. 2, for convenience, the extruder and the granule-forming chamber 6 are not shown in actual proportions. In the extruder of Fig. 2, the granule-forming chamber 6 is provided in such a manner that the discharge port 5b of the spray nozzle 5 is integrated inside the granule-forming chamber 6. The granule-forming chamber 6 has, at the bottom, a wax matrix granule collector 6a for collecting wax matrix granules that drop by gravity and accumulate at the bottom. The granule-forming chamber 6 has an exhaust member 7 at the side opposite to the discharge port 5b of the spray nozzle 5, and thus the spray air to be supplied to the chamber can be exhausted. The exhaust member 7 has an exhaust fan 7a and a collector 7b for collecting wax matrix granules in exhaust air. Thus, air in the granule-forming chamber 6 can be exhausted and wax matrix granules remaining in the exhausted air can be collected.

### Supply and melting-and-kneading of starting material

According to the method of the invention, a given amount of starting materials is supplied to the extruder, and the starting materials are melted and kneaded. The starting materials supplied to the extruder include ingredients to be contained in the drug-containing wax matrix granules to be produced, and at least one drug and at least one wax can be mentioned as specific examples. The drug-containing wax matrix granules produced by the invention may contain other additives in addition to the drug and the wax. When other additives are added, they are supplied to the extruder together with the drug and the wax as starting material.

The temperature of the barrel and die at the time that the starting materials are melted and kneaded by the extruder is higher than the melting point of the wax to be added, preferably higher by 5 to 200°C, and more preferably higher by 10 to 200°C, than the melting point of the wax to be added. The temperature of the barrel and the die must be adjusted in such a manner as not to adversely affect the stability of the drug, wax, and other additives to be added. The temperature of the barrel is preferably adjusted in such a manner that the temperature in the downstream side is in the above-mentioned range by gradually increasing the temperature from the upstream side (the side of the supplying port) to the downstream side (the side of the outlet die).

The period of time during which the supplied starting materials remain in the barrel, the screw rotation rate, and the starting material-supplying rate are determined in such a manner that the molten kneaded mixture of the starting materials is formed at least at the outlet die of the extruder.

### Spraying of the molten kneaded mixture of the starting materials and formation of granules

While the starting materials (drug, wax, and, as required, other additives) can be melted and kneaded under the above-described conditions, the molten kneaded mixture of the starting materials is sprayed and discharged from the spray nozzle, which is mounted on the die of the extruder, in an atmosphere having a temperature that is lower than the melting point of the wax. The discharge rate at which the molten kneaded mixture of the starting materials is discharged from the spray nozzle in an atmosphere having the above-mentioned temperature is determined in view of the particle diameter of the final drug-containing wax matrix granules, the viscosity of the molten kneaded mixture of the starting materials, the form of the spray nozzle, the opening diameter of the discharge port of the spray nozzle, and, in the case of a multi-fluid nozzle having at least two fluids, the spray air amount, etc.

For example, the discharge rate of the molten kneaded mixture of the starting materials from the extruder is usually 0.1 to 1000 kg per hour, preferably 0.5 to 700 kg per hour, and more preferably 1 to 400 kg per hour, per discharge port of a normal die. With the above-mentioned discharge rate, drug-containing wax matrix granules having a particle diameter in the range mentioned later can be produced. When the spray-air amount and the nozzle-opening diameter are the same, there is a tendency for a higher discharge rate to result in a larger particle diameter, and for a lower discharge rate to result in a smaller particle diameter. When spray air is not used, there is a tendency for a higher discharge rate to result in a smaller particle diameter, and for a lower discharge rate to result in a larger particle diameter.

The temperature of the air used for spraying the molten kneaded mixture of the starting materials is not limited. For example, in the case of a multi-fluid spray nozzle having at least two fluids, the temperature of the spray air is near or higher than the melting point of the wax to be added, preferably about -10 to +300°C thereof, more preferably about -10 to +250°C thereof, and still more preferably about ±0 to +200°C thereof.

The molten kneaded mixture of the starting materials discharged in an atmosphere having a temperature lower than the melting point of the wax under the above-described conditions is cooled in such an atmosphere to form spherical granules. The temperature of the atmosphere in which the molten kneaded mixture of the starting materials is discharged may be lower than the melting point of the wax and may be determined so that the molten kneaded mixture of the starting materials is solidified, and, for example, temperatures of -196 to 50°C, and preferably -196 to 40°C, can be mentioned. An atmosphere whose temperature is in the above-mentioned range can be prepared by a normal temperature control member, or may be produced using liquid nitrogen. A preferable embodiment is a method for spraying the molten kneaded mixture of the starting materials in a gas atmosphere having a temperature lower than the melting point of the wax.

Drug-containing wax matrix granules having a given particle diameter are produced by discharging the molten kneaded mixture of the starting materials in an atmosphere whose temperature is lower than the melting point of the wax, and then cooling the discharged substance under the above-described conditions. According to the production method of the invention, drug-containing wax matrix granules having an average particle diameter of 1.5 mm or less, preferably 0.01 to 1.5 mm, more preferably 0.02 to 1.0 mm, and still more preferably 0.03 to 0.9 mm can be produced. In the production method of the invention, the average particle diameter of the drug-containing wax matrix granules can be adjusted by appropriately adjusting the kind and amount of starting materials to be used, the discharge rate of the molten kneaded mixture of the starting materials, the spray air amount, etc. In this specification, the average particle diameter refers to a 50% cumulative diameter, and more specifically refers to a particle diameter when a volume integrated from 0 µm reaches 50% in a particle size distribution. The cumulative diameter is determined by a particle size distribution analyzer utilizing laser diffraction·scattering.

Prior-art techniques had a problem with piping blockage due to the recrystallization of the dissolve or melted drug in the interior of a pipe, a pipe connecting portion, or an atomizer portion. The problems of prior-art techniques can be solved by the production method of the invention. Therefore, the method of the invention is useful for producing spherical drug-containing wax matrix granules having an average particle diameter in the above-mentioned range.

### Formation of drug crystals in wax matrix granules

By forming crystals of a drug that is not completely formed into crystals in wax matrix granules, the drug can be imparted with a stable release controlling property.

To precipitate drug crystals having a desired crystal particle diameter, the drug-containing wax matrix granules obtained above can be stored at normal temperatures or heated. From the viewpoint of precipitating desired drug crystals in a short time, heating wax matrix granules is preferable.

It is preferable to adhere, prior to the heat-treatment, a predetermined amount of later-described inert particles to the surface of drug-containing wax matrix granules. By adhering the inert particles as mentioned above, the coagulation of wax matrix granules can be prevented and the production efficiency can be improved. Inert particles are easily adhered to the above-mentioned wax matrix granules by mixing inert particles with the wax matrix granules.

There is no limitation to the above-described heat-treatment conditions. The heating temperature is generally not less than room temperature and not more than the melting point of the wax, preferably 40 to 55°C, and more preferably 45 to 54°C. The heat treatment period varies according to the heat treatment temperature, and is usually 1 minute to 24 hours, preferably 5 minutes to 20 hours, and more preferably 10 minutes to 15 hours.

The above-described formation of drug crystals in the wax matrix granules is effective especially when cilostazol is used as the drug.

### Drug

There is no limitation on medical drugs usable in the production method of the invention insofar as they are pharmacologically acceptable and have a pharmacological action. Those that are water-soluble, fat-soluble, and water-insoluble may be used. Mentioned as one example of such medical drugs is a common medical drug to be added in various pharmaceutical preparations such as angiotensin II receptor antagonists (ARB), digestive agents, nutrient, alibility oil, opioid-based analgesic, calcium (Ca) antagonists, remedies for overactive bladder, keratin dissolving agents, cardiotonics, muscle relaxants, antineoplastic agents, antiviral agents, antiinflammatory drugs, antibacterial agents, antianginal drugs, anthelmintic, antidepressant drugs, schizophrenia treatment drugs, antiepileptic drugs, antiarrhythmic drugs, analgesic drugs, antifungal agents, anticoagulants, antidiabetic agents, antigout drugs, antihypertensives, anti-urinary incontinence agents, antimalarial drugs, antimigraine agents, antimuscarinic agents, antiparkinson agents, antihistamines, antiadipositacs, antianxiety drugs, antiarrhythmic drugs, anti-benign prostate hypertrophy agents, analeptic, remedies for osteoporosis, corticosteroid, CCR V receptor antagonist (HIV entry inhibitor), lipid modulators, anticonvulsants, erectile dysfunction treatment agents, immunosuppressant, antiprotozoals, antithyroid agents, Cox-2 inhibitor, hypnotics, muscle relaxants, sex hormone, sedatives, recognition enhancer, dysuria treatment agents, β blockers, essential fatty acids, non-essential fatty acids, protease inhibitors, macrolide antibiotic, diuretics, leukotriene antagonists, etc. In the invention, medical drugs may be used alone or in combination of two or more.

Specific examples of the medical drugs usable in the invention include acetretin, albendazole, aldbuterol, aminoglutethimide, amiodarone, amlodipine, amphetamine, amphotericin B, atorvastatin, atovaquone, azithromycin, baclofen, beclomethasone, benazepril, benzonatat, betamethason, bicalutamide, budesonide, bupropion, busulfan, butenafine, calcifediol, calcipotriene, calcitrio, camptothecin, candesartan, capsaicin, carbamazepine, carotene, celecoxib, cerivastatin, cetirizine, chlorpheniramine, cholecalciferol, cilostazol, cimetidine, cinnarizin, ciprofloxacin, cisapride, clarithromycin, clemastine, clomiphene, clomipramine, clopidogrel, codeine, coenzyme Q10, cyclobenzaprine, cyclosporine, danazol, dantrolene, dexychlorpheniramine, diclofenac, dicumarol, digoxin, dehydroepiandrosterone, dihydroergotamine, dihydrotachysterol, dirithromycin, donepezil, efavirenz, eprosartan, ergocalciferol, ergotamine, source of essential fatty acid, etodolac, etoposide, famotidine, fenofibrate, fentanyl, fexofenadine, finasteride, fluconazole, flurbiprofen, fluvastatin, fosphenytoin, fiovatriptan, futazolidone, gabapentin, gemfibrozil, glibenclamide, glipizide, glyburide, glimepiride, griseofulvin, halofantrine, ibuprofen, irbesartan, irinotecan, isosorbide dinitrates, isotretinoin, itraconazole, ivermectin, ketoconazole, ketorolac, lamotrigine, lansoprazole, leflunomide, lisinopril, loperamide, loratadine, lovastatin, L-thyroxine, lutein, lycopene, medroxyprogesterone, mifepristone, mefloquine, megestrol acetate, methadone, methoxsalen, metronidazole, miconazole, midazolam, miglitol, minoxidil, mitoxantrone, montelukast, nabumetone, nalbuphine, nelfinavir, nifedipine, nisoldipine, nilutanide, nitrofurantoin, nizatidine, omepurazor, oprelvekin, estradiol, oxaprozin, paclitaxel, paracalcitol, paroxetine, pentazocine, pioglitazone, pizotifen, pravastatin, prednisolone, probucol, progesterone, pseudoephidrene, pyridostigmine, rabeprazol, raloxifene, rofecoxib, repaglinide, rifabutin, rifapentine, rimexolone, ritanovir, rizatriptan, rosiglitazone, saquinavir, sertraline, sibutramine, sildenafil citrate, simvastatin, sirolimus, spironolactone,sumatriptan, tacrin, tacrolimus, tomoxifen, tamsulosin, targretin, tazarotene , telmisartan, teniposide, terbinafine, terazosin, tetrahydrocannabinol, tiagabine, ticlopidine, tirofiban, tizanidine, topiramate, topotecan, toremifene, tramadol, tretinoin, troglitazone, trovafloxacin, ubidecarenone, valsartan, venlafaxine, verteporfin, vigabatrin, vitamin A, vitamin D, vitamin E, vitamin K, zafirlukast, zileuton, zolmitriptan, zolpidem, zopicione, acarbose, acyclovir, acetylcysteine, acetylcholine chloride, alatrofloxacin, alendronate, alglucerase, amantadine hydrochloride, ambenonium, amifostine, amiloride hydrochloride, aminocaproic acid, amphotericin B, aprotinin, asparaginase, atenolol, atracurium besilate, atropine, azithromycin, aztreonam, BCG vaccine, bacitracin, becalermin, belladonna, bepridil hydrochloride, bleomycin sulfate, human calcitonin, salmon calcitonin, carboplatin, capecitabine, capreomycin sulfate, cefamandole nafate, cefazolin sodium, cefepime dihydrochloride, cefixime, cefonicid sodium, cefoperazone, cefotetan disodium, cefotaxime, cefoxitin sodium, ceftizoxime, ceftriaxone, cefuroxime axetil, cephalexin, cephapirin sodium, cholera vaccine, cidofovir, cisplatin, cladribine, clidinium bromide, clindamycin, clindamycin derivatives, ciprofloxacin, clodronate, colistimethate sodium, colistin sulfate, corticotropin, cosyntropin, cromolyn sodium, cytarabine, dalteparin sodium, danaparoid, deferoxamine, denileukin diftitox, desmopressin, diatrizoate meglumine, diatrizoate sodium, dicyclomine, didanosine, dirithromycin, dopamine hydrochloride, deoxyribonuclease α, doxacurium chloride, doxorubicin, etidronate disodium, enalaprilat, enkephalin, enoxacin, enoxaparin sodium, ephedrine, epinephrine, erythromycin, esmolol hydrochloride, famciclovir, fludarabine, fluoxetine, oscarnet sodim, ganciclovir, gentamycin, glucagon, glycopyrrolate, gonadorelin, indinavir sulfate, influenza virus vaccines, ipratropium bromide, ifosfamide, lamivudine, leucovorin calcium, leuprolide acetate, levofloxacin, lincomycin, lincomycin derivatives, lobucavir, lomefloxacin, loracarbef, mepenzolate bromide, mesalamine, methenamine, methotrexate, methscopolamine, metformin hydrochloride, metoprolol, mezlocillin sodium, mivacurium chloride, nedocromil sodium, neostigmine bromide, neostigmine methylsulfate, neurontin, norfloxacin, octreotide acetate, ofloxacin, olpadronate, oxytocin, pamidronate disodium, pancuaronium bromide, paroxetine, perfloxacin, pentamidine isetionate, pentostatin, pentoxifylline, penciclovir, pentagastrin, phentolamine mesylate, phenylalanine, physostigmine salicylate, plague vaccine, piperacillin sodium, polymyxin B sulfate, pralidoxime chloride, pramlintide, pregabalin, propafenone, propantheline bromide, pyridostigmine bromide, rabies vaccine, risedronate, ribavirin, rimantadine hydrochloride, salmeterol xinafoate, sincalide, solatol, somatostatin, sparfloxacin, spectinomycin, stavudine, streptokinase, streptozocin, suxamethonium chloride, tacrine hydrochloride, terbutaline sulfate, thiopeta, ticarcillin, tiludronate, timolol, trandolapril, trimetrexate gluconate, trospectinomycin, trovafloxacin, tubocurarine chloride, urea, urokinase, vancomycin, valacyclovir, valsartan, vasopressin, vasopressin derivatives, vecuronium bromide, vinblastine, vincristine, vinorelbine, Vitamin B12, warfarin sodium, zalcitabine, zanamivir, zolandronate, zidovudine, theophylline, grepafloxacin, carteolol, procaterol, rebamipide, aripiprazole, tolvaptan, acetaminophen, ketoprofen, naproxen, piroxicam, phenytoin, verapamil, pharmacologically acceptable salts thereof, isomers thereof, derivatives thereof, etc.

Since drug-containing wax matrix granules can be imparted with sustained-release property, drugs that require a sustained-release property are preferable in the invention. From such a viewpoint, preferable as drugs for use in the invention are theophylline, cilostazol, ketoprofen, naproxen, diclofenac, itraconazole, piroxicam, phenytoin, verapamil, probucol, tolvaptan. Among these, theophylline, cilostazol, probucol, and tolvaptan are more preferable.

Even in the case of using a drug whose crystals are likely to precipitate out, the invention makes it possible to produce drug-containing wax matrix granules while avoiding the precipitation of the drug crystals when the base ingredient (wax) is dissolving or melting. Considering this effect of the invention, drugs whose crystals are likely to precipitate when the base ingredient (wax) is melting (e.g., drugs whose melting point is about 100 to about 200°C) can be mentioned as preferable examples of a drug for use in the invention. One such example is cilostazol.

The drug content in the drug-containing wax matrix granules produced according to the invention differs according to the kind and action of the drug used, and the gender and age of the person to be treated, etc. For example, the content is 0.001 to 90% by weight, preferably 0.05 to 95% by weight, and more preferably 0.1 to 90% by weight, based on the total of wax matrix granules.

### Wax

There is no limitation on the wax matrix base material to be used in the production method of the invention insofar as it is pharmacologically acceptable and is in a solid form at a normal temperature (30°C). For example, waxes from animal fat, waxes from vegetable fat, synthetic waxes, or semi-synthetic waxes whose melting point ranges from 40 to 120°C, and preferably from 40 to 90°C, may be used. The melting point is determined according to "the general test procedures described in the Japanese Pharmacopoeia 14th Edition; 14. Congealing point Determination".

Specific examples of waxes include paraffin, micro crystallin wax, ceresin, Japan wax, cacao butter, carnauba wax, beeswax, cetanol, steryl alcohol, myristic acid, palmitic acid, stearic acid, glycerine fatty acid ester, polyglycerin fatty acid ester, glycerin organic-acid fatty acid ester, propylene glycol fatty acid ester, sorbitan fatty acid ester, hydrogenated oil, etc.

Glycerine fatty acid esters are monoesters, diesters, and triesters of glycerin and various fatty acids. Mentioned as fatty acids of such glycerine fatty acid esters are C₆₋₂₂ fatty acids. Examples of such fatty acids include stearic acids, behenic acids, palmitic acids, oleic acids, linoleic acids, linolenic acids, myristic acids, lauric acids, ricinoleic acids, caprylic acids, capric acids, etc. Examples of glycerol fatty acid esters include glycerin monostearate, glycerol distearate, glycerol tristearate, glycerol monobehenate, glycerol dibeherate, glycerol tribehenate, glycerol monostearate monobehenate, etc.

Polyglycerol fatty acid esters refer to esters in which one or more fatty acids are bonded to polyglycerol in which two or more glyrecols are polymerized. Such polyglycerol fatty acid esters include polyglycerol full esters of fatty acid in which fatty acid is linked via an ester bond to all of the hydroxyl groups of polyglycerol, polyglycerol half esters of fatty acid in which fatty acid is linked via an ester bond to half of the hydroxyl groups of polyglycerol, and the like. The "polyglycerol half ester of fatty acid" refers to a polyglycerol fatty acid ester or a mixture thereof in which the average number (N_{E}) of esterified hydroxyl groups in the polyglycerol is about the half of the number (N) of hydroxyl groups present in the non-esterified polyglycerol. Such polyglycerol half esters of fatty acid have 0.3≤N_{E}/N≤0.7, and preferably 0.35≤N_{E}/N≤0.65. For example, a triglycerol half ester of behenic acid means an ester in which 2 or 3 behenic acids are ester-linked to a triglycerol having 5 hydroxyl groups, the triglycerol being 3 glycerol molecules dehydratively condensed, or a mixture thereof, i.e., triglycerol behenic acid (di or tri) ester.

Fatty acids of polyglycerol fatty acid esters are, for example, C₆₋₂₂ fatty acids, and specific examples of such fatty acids are the same as with the above-mentioned fatty acids of glycerol fatty acid esters. Specific examples of polyglycerol fatty acid esters include diglycerol mono- or di-stearate; diglycerol mono- or dipalmitate; diglycerol-mono or di-laurate; diglycerol mono- or di-oleate; diglycerol mono- or di-linolate; diglycerol mono- or di-caprylate; diglycerol mono- or di-behenate; triglycerol mono-, di-, tri-, tetra-, or penta-stearate; triglyrecol mono-, di-, tri-, tetra-, or penta-palmitate; triglyrecol mono-, di-, tri-, tetra-, or penta-laurate; triglyrecol mono-, di-, tri-, tetra-, or penta- oleate; triglyrecol mono-, di-, tri-, tetra-, or penta- linolate; triglyrecol mono-, di-, tri-, tetra-, or penta- caprylate; triglyrecol mono-, di-, tri-, tetra-, or penta- behenate; tetraglyrecol mono-, di-, tri-, tetra-, penta-, or hexa stearate; tetraglyrecol mono-, di-, tri-, tetra-, penta-, or hexa palmitate; tetraglyrecol mono-, di-, tri-, tetra-, penta-, or hexa laurate; tetraglyrecol mono-, di-, tri-, tetra-, penta-, or hexa oleate; tetraglyrecol mono-, di-, tri-, tetra-, penta-, or hexa linolate; tetraglyrecol mono-, di-, tri-, tetra-, penta-, or hexa caprylate; tetraglyrecol mono-, di-, tri-, tetra-, penta-, or hexa behenate; pentaglycerolmono-, di-, tri-, tetra-, penta-, hexa-, or penta- stearate; pentaglycerol mono-, di-, tri-, tetra-, penta-, or hexa- palmitate; pentaglycerol mono-, di-, tri-, tetra-, penta-, or hexa- laurate; pentaglycerol mono-, di-, tri-, tetra-, penta-, or hexa- oleate; pentaglycerol mono-, di-, tri-, tetra-, penta-, or hexa- linolate; pentaglycerol mono-, di-, tri-, tetra-, penta-, or hexa- caprylate; pentaglycerol mono-, di-, tri-, tetra-, penta-, or hexa- behenate; hexaglycerolmono-, di-, tri-, tetra-, penta-, hexa-, or hepta stearate; hexaglycerol mono-, di-, tri-, tetra-, penta-, hexa-, or hepta palmitate; hexaglycerol mono-, di-, tri-, tetra-, penta-, hexa-, or hepta laurate; hexaglycerol mono-, di-, tri-, tetra-, penta-, hexa-, or hepta oleate; hexaglycerol mono-, di-, tri-, tetra-, penta-, hexa-, or hepta linolate; hexaglycerol mono-, di-, tri-, tetra-, penta-, hexa-, or hepta caprylate; hexaglycerol mono-, di-, tri-, tetra-, penta-, hexa-, or hepta behenate; heptaglycerol mono-, di-, tri-, tetra-, penta-, hexa-, hepta, or octa stearate; heptaglycerol mono-, di-, tri-, tetra-, penta-, hexa-, hepta, or octa palmitate; heptaglycerol mono-, di-, tri-, tetra-, penta-, hexa-, hepta, or octa laurate; heptaglycerol mono-, di-, tri-, tetra-, penta-, hexa-, hepta, or octa oleate; heptaglycerol mono-, di-, tri-, tetra-, penta-, hexa-, hepta, or octa linolate; heptaglycerol mono-, di-, tri-, tetra-, penta-, hexa-, hepta, or octa caprylate; heptaglycerol mono-, di-, tri-, tetra-, penta-, hexa-, hepta, or octa behenate; decaglycerol mono-, di-, tri-, tetra-, penta-, hexa-, hepta-, octa-, nona-, deca-, or undeca-stearate; decaglycerol mono-, di-, tri-, tetra-, penta-, hexa-, hepta-, octa-, nona-, deca-, or undeca- palmitate; decaglycerol mono-, di-, tri-, tetra-, penta-, hexa-, hepta-, octa-, nona-, deca-, or undeca- laurate; decaglycerol mono-, di-, tri-, tetra-, penta-, hexa-, hepta-, octa-, nona-, deca-, or undeca- oleate; decaglycerol mono-, di-, tri-, tetra-, penta-, hexa-, hepta-, octa-, nona-, deca-, or undeca- linoleate; decaglycerol mono-, di-, tri-, tetra-, penta-, hexa-, hepta-, octa-, nona-, deca-, or undeca- caprylate; decaglycerol mono-, di-, tri-, tetra-, penta-, hexa-, hepta-, octa-, nona-, deca-, or undeca- behenate, etc. In addition to these, polyglycerol fatty acid esters comprising polyglycerol esters and two or more fatty acids of stearic acid, behenic acid, palmitic acid, oleic acid, linoleic acid, linolenic acid, myristic acid, lauric acid, ricinoleic acid, caprylic acid, and capric acid.

Glycerol organic acid fatty acid esters refer to esters in which organic acid(s) and fatty acid(s) are bonded to glycerol. C₆₋₂₂ fatty acids are examples of fatty acids of such glycerol organic acid fatty acid esters. Specific examples of such fatty acids are the same as with the above-mentioned fatty acids of glycerol fatty acid esters. Specific examples of glycerol organic acid fatty acid esters are glycerol citric acid fatty acid esters, glycerol acetic acid fatty acid esters, glycerol lactic acid fatty acid esters, glycerol succinic acid fatty acid esters, glycerol fumaric acid fatty acid esters, glycerol tartaric acid fatty acid esters, glycerol diacetyl tartaric acid fatty acid esters, polyglycerol citric acid fatty acid esters, polyglycerol acetic acid fatty acid esters, polyglycerol lactic acid fatty acid esters, polyglycerol succinic acid fatty acid esters, polyglycerol fumaric acid fatty acid esters, polyglycerol tartaric acid fatty acid esters, polyglycerol diacetyl tartaric acid fatty acid esters, etc.

C₆₋₂₂ fatty acids are examples of fatty acids of sorbitan fatty acid esters. Specific examples such fatty acids are the same as with the above-mentioned fatty acids of glycerol fatty acid esters. Specific examples of sorbitan fatty acid esters are sorbitan laurate, sorbitan palmitate, sorbitan oleate, sorbitan stearate, etc.

C₆₋₂₂ fatty acids are examples of fatty acids of propylene glycol fatty acid esters. Specific examples such fatty acids are the same as with the above-mentioned fatty acids of glycerol fatty acid esters. Specific examples of propylene glycol fatty acid esters are propylene glycol myristate, propylene glycol stearate, propylene glycol laurate, propylene glycol oleate, propylene glycol caprylate, etc. In addition to these, propylene glycol fatty acid esters in which two or more fatty acids are incorporated are mentioned.

Specific examples of hydrogenated oils include castor oil, cottonseed oil, soybean oil, rapeseed oil, beef tallow, etc.

Among the above-mentioned waxes, glycerol fatty acid esters and polyglycerol fatty acid esters are preferable.

The above-mentioned waxes may be used singly or in combination.

The wax content of the drug-containing wax matrix granules to be produced is, for example, 0.1 to 99.99% by weight, preferably 0. 5 to 99% by weight, and more preferably 1 to 90% by weight based on the total amount of wax matrix granules.

### Ingredients to be added as required (Additives)

In the production method of the invention, a suitable amount of surfactant can be further added as starting material in addition to the above-mentioned drugs and waxes. Examples of such surfactants include alkylglucosides, alkyl maltosides, alkylthioglucosides, lauryl macrogol glycerides, polyoxyethylene alkyl ethers, polyoxyethylene alkyl phenols, polyethylene glycol fatty acid esters, polyethylene glycol glycerol fatty acid esters, polyethylene sorbitan fatty acid esters, polyoxyethylene polyoxypropylene alkyl ethers, polyoxyethylene polyoxypropylene block copolymers, polyoxyethylene glycerides, polyoxyethylene sterols, derivatives thereof, polyoxyethylene vegetable oils, polyoxyethylene hydrogenated vegetable oils, tocopherol polyethylene-glycols succinat (TPGS), sugar esters, sugar ethers, sucroglycerides, esters of fatty acids (C₈₋₁₈) and lower alcohols (C₂₋₄), or the like.

Specific examples of the above-mentioned surfactants include polyoxyethylene lauryl ethers, polyoxyethylene cetyl ethers, polyoxyethylene stearyl ethers, polyoxyethylene oleyl ethers, polyoxyethylene behenyl ethers, and like polyoxyethylene alkyl ethers; polyethylene glycol laurates, polyethylene glycol stearates, polyethylene glycol oleates, polyethylene glycol palmitates, mixtures of polyethylene glycol fatty acid mono- and di-esters, and like polyethylene glycol esters; polyethylene glycol glyceryl laurates, polyethylene glycol glycerol stearates, polyethylene glycol glycerol oleates, and like polyethylene glycol glycerol fatty acid esters; polyoxyethylene phytosterols, polyoxyethylene cholesteryl esters, polyoxyethylene cholestanol esters, and like polyoxyethylene sterols and derivatives thereof; polyethylene glycol sorbitan laurates, polyethylene glycol sorbitan oleates, polyethylene glycol sorbitan palmitates, and like polyethylene glycol sorbitan fatty acid esters; polyoxyethylene polyoxypolypropylene cetyl ethers, polyoxyethylene polyoxypolypropylene decyl tetradecyl ethers, and like polyoxyethylene polyoxypolypropylene alkyl ethers; poloxamer 105, 108, 122, 123, 124, 181, 182, 183, 184, 185, 188, 212, 215, 217, 231, 234, 235, 237, 238, 282, 284, 288, 331, 334, 335, 338, 401,402,403,407, etc., Pluronic (registered trademark) series (BASF), Emkalyx, Lutrol (BASE), Supronic, Monolan, Pluracare, Plurodac, and like polyoxyethylene-polyoxypropylene block copolymers; sucrose mono- or di- stearate, sucrose mono- or di-palmitate, sucrose mono- or dilaurate, and like sugar esters; ethyl oleate, isopropyl myristate, isopropyl palmitate, ethyl linolenate, isopropyl linolenate, and like esters of lower alcohols (C₂₋₄) and fatty acids (C₈₋₁₈), etc.

In the production method of the invention, a suitable amount of polymer can be added. Examples of polymers include water soluble polymers that are soluble or dispersible in a molten kneaded wax, water insoluble polymers, enteric polymers, gastric juice soluble polymers, etc. Specific examples of such polymers include hydroxy cellulose, hydroxypropylmethylcellulose, hydroxypropylmethylcellulose acetate succinate, cellulose acetate phthalate, ethylcellulose, cellulose acetate, polyvinylpyrrolidone, hydroxyethyl cellulose, methylcellulose, hydroxypropylmethylcellulose phthalate, carmellose, carmellose sodium, hydroxyethyl cellulose, cyclodextrin, cyclodextrin derivatives, amino alkyl methacrylate copolymer E, alkyl methacrylate copolymer RS, methacrylate copolymer L, methacrylate copolymer S, carboxy vinyl polymer, polyvinyl acetal diethyl amine acetate, polyvinyl alcohol, sodium alginate, propylene glycol alginate, gelatin, shellac, etc.

Examples of additives which can be added as starting material besides the above, inert particles, ion exchange resins, solubilizers, plasticizers, diluents, sweetners, lubricants, carriers or fillers, enzyme inhibitors, anti-adhesives, anticoagulants, defoaming agents, binders, pH adjusters or buffers, chelating agents, coagulants, absorption enhancers, binders, desensitizers, flavors, preservatives, antioxidant, antifreezes, colorants, opaquers, coolants, solvents, thickeners, disintegrators, etc. Specific examples of such additives include lecithin, lysolecithin, phosphatidylcholine, phosphatidylethanolamine, phosphatidylglycerol, phosphatidic acid, phosphatidylserine, lysophosphatidylcholine, lysophosphatidylethanolamine, lysophosphatidylglycerol, lysophosphatidic acid, lysophosphatidylserine, PEG-phosphatidylethanolamine, PVP-phosphatidylethanolamine, lactate ester of fatty acid, steayl-2-lactate, steayl-lactate, succinylated monoglyceride, mono- / di- acetylized tartrate ester of mon- /di- glyceride, citrate ester of mon- /di- glyceride, cholic acid, taurocholic acid, glycocholic acid, deoxycholic acid, taurodeoxycholic acid, chenodeoxycholic acid, glycodeoxycholic acid, glycochenodeoxycholic acid, taurchenodeoxycholic acid, ursodeoxycholic acid, tauroursodeoxycholic acid, glycoursodeoxycholic acid, cholic acid sarcosine, N-methyl taurocholic acid, caproic acid, caprylic acid, capric acid, lauric acid, oleic acid, ricinoleic acid, linoleic acid, linolenic acid, lauryl sulfate, teracecyl sulfate, docusate, lauroryl carnitine, palmitoyl carnitine, myristoyl carnitine, sodium caprate, sodium caprylate, sodium laurate, sodium myristate, sodium myristolate, sodium palmitate, sodium palmitoate, sodium oleate, sodium lithocholate, sodium linolate, sodium linolenate, sodium stearate, sodium dodecyl sulfate, sodium tetradecyl sulfate, sodium lauryl sarcosinate, sodium taurocholate, sodium glycolate, sodium deoxycholate, sodium taurodeoxycholate, sodium glycodeoxycholate, sodium ursodeoxycholate, sodium chenodeoxycholate, cardiolipin, macrogol 400, macrogol 4000, macrogol 600, macrogol 10000, macrogol 6000, lactose, saccharose, mannitol, sodium chloride, glucose, calcium carbonate, kaolin, crystalline cellulose, cellulose-based polymers, light anhydrous silicic acid, silicate, water, ethanol, simple syrup, glucose liquid, starch liquid, gelatin liquid, dextrin, pullulan, citric acid, anhydrous citric acid, sodium citrate, sodium citrate dihydrate, anhydrous sodium monohydrogenphosphate, anhydrous sodium dihydrogenphosphate, sodium phosphate, polysorbate 80, quaternary ammonium salt group, sodium lauryl sulfate, purified talc, stearate, polyethylene glycol, colloid-like silicic acid, yellow iron oxide, yellow 32 iron oxide, 32 iron oxide, β carotene, titanium oxide, food colorant (e.g., food blue No.1, and the like), copper chlorophyll, riboflavin, ascorbic acid, aspartame, hydrangeae dulcis folium, sodium chloride, fructose, saccharin, powdered sugar, etc.

The above-mentioned additive (s) may be supplied as starting material to the extruder together with the above-mentioned drug (s) and wax (es), or mixed with previously-formed wax matrix granules.

### Drug-containing wax matrix granules

The drug-containing wax matrix granules produced by the method of the present invention are used for a preparation. The preparation may be used in the form of a powder or granular preparation consisting of the drug-containing wax matrix granules and can be used in the form of a capsule in which the drug-containing wax matrix granules are filled into microcapsules, soft capsules, hard capsules, etc.

### 2. Cilostazol-containing sustained-release preparation

The present invention also provides a sustained-release preparation comprising cilostazol-containing wax matrix granules. The cilostazol-containing wax matrix granules contained in the sustained-release preparation can be easily produced by the above-mentioned production method, and may also be produced by other production methods without limitation.

The sustained-release preparation of the present invention contains cilostazol crystals (hereafter sometimes referred to simply as "ingredient (A)") as a drug. The average particle diameter of the cilostazol crystals is not limited, and may be, for example, 10 µm or less, preferably 0.1 to 10 µm, and more preferably 0.5 to 8 µm. The use of cilostazol in the form of crystals having the above average particle diameter makes it possible to more stably perform sustained release and absorption of cilostazol in the lower part of the gastrointestinal tract, in which the water content is low.

Cilostazol crystals with the above average particle diameter can be produced by allowing wax matrix granules in which cilostazol has been completely dissolved or melted, to remain untouched at room temperature, but when wax matrix granules in which cilostazol has been completely dissolved or melted are subjected to a heat treatment, cilostazol crystals having the above average particle diameter can be produced more rapidly than by allowing them to remained untouched at room temperature. Specifically, cilostazol crystals with the above average particle diameter can be produced in the wax matrix granules by mixing and heating predetermined amounts of cilostazol and the ingredient (B) mentioned hereinafter, solidifying the obtained molten mixture into particles, and then heating the particles at a temperature not lower than room temperature and not higher than the melting point of the ingredient (B), preferably 40 to 55°C, and more preferably 45 to 54°C. The heat treatment time is not limited, and may be, for example, 1 minute to 24 hours, preferably 5 minutes to 20 hours, and more preferably 10 minutes to 15 hours.

The average particle diameter of cilostazol crystals is measured by observation with a polarizing microscope. Specifically, the measurement is made by indicating a ruler with a predetermined size in the field of vision under a polarizing microscope to thereby observe the size of the crystals.

In the sustained-release preparation of the present invention, the concentration of the ingredient (A) varies depending on the intended use of the preparation, the gender and age of the subject to whom the preparation is to be administered, etc., and may be, for example, 5 to 60 wt.%, preferably 10 to 50 wt.%, and more preferably 20 to 45 wt.%, relative to the total amount of the wax matrix granules contained in the preparation.

Further, the sustained-release preparation of the present invention contains, in addition to the above-mentioned ingredient (A), glycerol fatty acid ester (s) and/or polyglycerol fatty acid ester (s) (hereinafter simply referred to as "ingredient (B)") as a wax(s) (wax matrix base material(s)).

Glycerol fatty acid esters and polyglycerol fatty acid esters as mentioned above are usable.

Such ingredients (B) can be used singly or in combination.

Among such ingredients (B), from the viewpoint of improving the sustained-release property and reducing the influence of meals on the cilostazol release rate, glycerol behenate, diglycerol stearate, triglycerol half-ester of behenic acid, triglycerol half-ester of stearic acid and decaglycerol monostearate are preferable, and glycerol behenate, diglycerol stearate, and half ester of triglycerol with behenic acid are particularly preferable.

In the sustained-release preparation of the present invention, the proportion of ingredients (A) and (B) is not limited, but the proportion of ingredient (B) is usually 50 to 2000 parts by weight, preferably 70 to 1000 parts by weight, and more preferably about 100 to 500 parts by weight, per 100 parts by weight of ingredient (A). The use of the ingredients in the above proportions more effectively improves the sustained release of cilostazol, and makes it unlikely that the release property will be influenced by meals.

In the sustained-release preparation of the present invention, the concentration of ingredient (B) can be selected according to the proportion of ingredients (A) and (B) and the amount of ingredient (A) described above. For example, the concentration of ingredient (B) may be 30 to 95 wt.%, preferably 40 to 90 wt.%, and more preferably 50 to 80 wt.%, relative to the total amount of the wax matrix granules contained in the preparation.

The sustained-release preparation of the present invention may further contain (C) a water-soluble cellulose derivative (water-soluble cellulose ether) such as hydroxypropylmethylcellulose, hydroxypropylmethylcellulose acetate succinate, cellulose acetate phthalate, cellulose acetate, polyvinylpyrrolidone,hydroxyethyl cellulose, methylcellulose, hydroxypropylmethylcellulose phthalate and the like, in addition to the ingredients (A) and (B). Among these, hydroxypropylcellulose and hydroxypropylmethylcellulose are preferable, and hydroxypropylmethylcellulose is more preferable. The above-mentioned water-soluble cellulose derivatives may be used singly or in combination. The use of a water-soluble cellulose derivative imparts high bioavailability while maintaining a sustained-release property. When a water-soluble cellulose derivative is contained in the sustained-release preparation of the present invention, its proportion may be, for example, 1 to 15 wt.%, preferably 2 to 12 wt. %, and more preferably 2 to 10 wt.% relative to the total amount of the wax matrix granules contained in the preparation.

The sustained-release preparation of the present invention can further contain a surfactant. Usable surfactants are the same as mentioned in "1. Production method for drug-containing wax matrix granules".

Further, the sustained-release preparation of the invention can contain a suitable amount of polymers, such as water-soluble polymers, water-insoluble polymers, enteric polymers, gastric juice-soluble polymers, etc. Specific examples of these polymers include those exemplified in the item "1. Method for producing drug-containing wax matrix granules" above.

Furthermore, in addition to the above, the sustained-release preparation of the invention can contain a suitable amount of additives such as inert particles, ion exchange resins, solubilizers, plasticizers, diluents, sweeteners, lubricants, carriers or fillers, enzyme inhibitors, anti-adhesives, anticoagulants, defoaming agents, binders, pH adjusters or buffers, chelating agents, coagulants, absorption enhancers, desensitizers, corrigents, preservatives, anti-oxidization agents, antifreezing agents, colorants, opaquers, coolants, solvents, thickeners, disintegrators, etc. Specific examples of these additives are the same as mentioned in "1. Production method for drug-containing wax matrix granules".

The above ingredients other than ingredients (A) and (B) may be contained in wax matrix granules together with the above ingredients (A) and (B), or may be contained by being mixed with wax matrix granules that already contain ingredients (A) and (B).

Among the above-mentioned additives, inert particles exhibiting no chemical or biological activity may be contained in such a manner that they cover the surface of wax matrix granules containing the above ingredients (A) and (B). Covering the surface of wax matrix granules with inert particles is useful for inhibiting the agglomeration of granules during the heat treatment for the cilostazol crystal formation.

Specific examples of the above inert particles include talc; light anhydrous silicic acid; titanium oxide; hydroxypropyl methylcellulose, hydroxypropylcellulose, hydroxypropyl methylcellulose phthalate, ethylcellulose, and like cellulose-based polymers; fructose, saccharine, powdered sugar, and like saccharides, etc. These inert particles can be used singly, or any two or more can be used in combination. The average particle diameter of the above-mentioned inert particles is not limited, but, for example, can be 10 µm or less, and preferably from 7 to 10 µm. The average particle diameter of these inert particles can be measured using methods commonly employed for measuring particle diameter powders.

The adhesion amount of these inert particles is not limited, but may be adhered in a total amount of 0.5 to 15 parts by weight, preferably 1 to 10 parts by weight, and more preferably 2 to 10 parts by weight, per 100 parts by weight of the wax matrix granules containing ingredients (A) and (B).

The wax matrix granules to be contained in the sustained-release preparation of the present invention are preferably those prepared by mixing certain amounts of the above ingredients (A) and (B), and, if required, other ingredients, heating the mixture to obtain a molten mixture, sizing the molten mixture to the desired particle diameter, and solidifying the granules. More preferable granules to be contained in the sustained-release preparation of the present invention are those produced by the method described in "1. Production method for drug-containing wax matrix granules" using ingredients (A) and (B), and, if required, other ingredients.

The wax matrix granules to be contained in sustained-release preparation of the present invention have an average particle diameter ranging from 40 to 200 µm. The average particle diameter is preferably ranging from 50 to 150 µm, and more preferably from 60 to 130 µm. Having the average particle diameter within these ranges and containing the above ingredients (A) and (B) in combination enable the desired sustained-release property of cilostazol to be exhibited and moderate the influence of food intake on the cilostazol release property. The average particle diameter used herein refers to a 50% cumulative diameter, i.e., the particle diameter when a volume integrated from 0 µm reaches 50% in a particle distribution, and the value measured with a particle size distribution analyzer utilizing laser diffraction scattering.

The wax matrix granules contained in the sustained-release preparation of the present invention can provide a good sustained-release property by containing the above-described ingredients (A) and (B), and average particle diameter. The sustained-release property of the granules contained in the sustained-release preparation of the present invention is not limited, but when tested using granules in an equivalent amount of 15 mg of cilostazol in accordance with Method 2 (the paddle method) in the dissolution test described in the Japanese Pharmacopoeia 14th Edition, the dissolution rate is preferably 20 to 35% 2 hours after dissolution, 40 to 60% 6 hours after dissolution, 60 to 80% 12 hours after dissolution, and 60 to 90% 18 hours after dissolution; and more preferably 25 to 35% 2 hours after dissolution, 45 to 60% 6 hours after dissolution, 60 to 80% 12 hours after dissolution, and 65 to 90% 18 hours after dissolution.

The sustained-release preparation of the present invention may be in the forms of a powder or granular preparation of the wax matrix granule themselves containing the above-mentioned ingredients (A) and (B), but may also be in the form of an encapsulated formulation in which the wax matrix granules are inserted into microcapsules, soft capsules, hard capsules, etc.

The dosage of the sustained-release preparation of the present invention may be selected according to the intended pharmaceutical use, the age and gender of the patient, etc.

### EXAMPLES

The present invention will be described in detail according to Examples, but is not limited thereto.

### Example 1

Wax matrix granules were produced using an extruder configured as shown in Fig. 2. The configuration and operation conditions of the extruder were as follows:
Extruder type: twin screw extruder (KEX-25, manufacture by Kurimoto)
Screw form: a conveyor member, a kneading member, and a mixing member are connected in series from the downstream side to the upstream side
Spray nozzle: two-fluid nozzle
Screw length: about 50 cm
Screw rotation rate: 125 rpm
Form and opening diameter of discharge port of spray nozzle:
   circular, φ 0.5 mm
   Period of time in which starting materials remained in a barrel: about two minutes
Barrel temperatures:
   140°C for a barrel jacket 1a-1
   150°C for a barrel jacket 1a-2
   160°C for barrel jackets 1a-3 and 1a-4
Spray air temperature and introduction rate:
   about 200°C, 25 L/min,
   Molten kneaded mixture of starting materials discharging rate per discharge port: about 50 g/minute
Atmosphere inside a granule-forming chamber:
   air, about 30°C

Specifically, 300 g of theophylline and 700 g of glycerol fatty acid ester (glycerol monobehenate; melting point of about 75°C) were mixed. While the resulting mixture of the starting materials was supplied to a supply port of the above-mentioned extruder at about 50 g/min, wax matrix granules were produced by an extruder configured as described above and having the above-described conditions, and the wax matrix granules produced were then collected from a wax matrix granule collector of the granule-forming chamber.

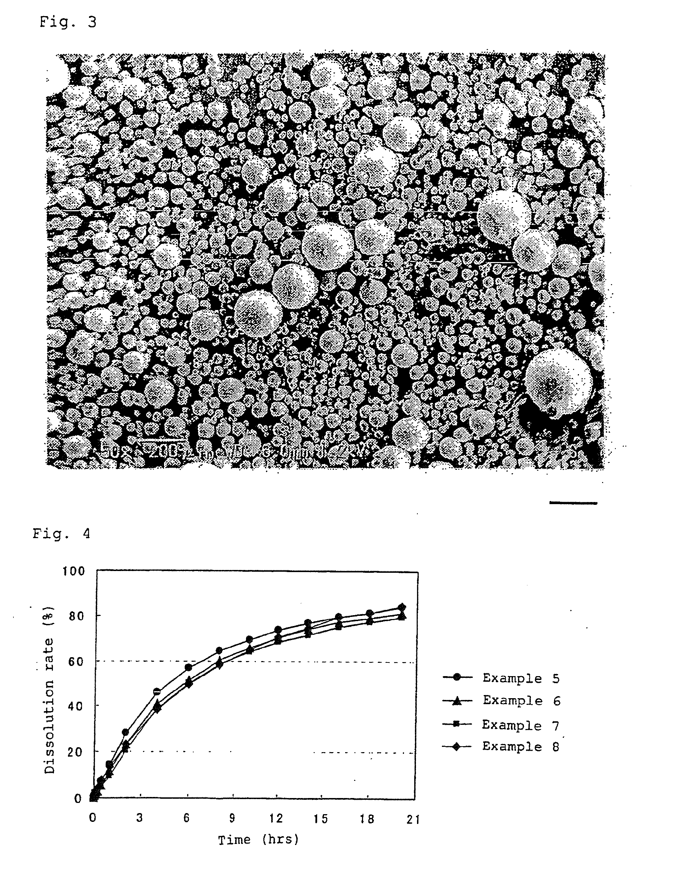

The obtained wax matrix granules were observed under a microscope, and the results are shown in Fig. 3. The obtained wax matrix granules were spherical. The particle size distribution was measured with a laser diffraction type particle size distribution analyzer (Tohnichi Computer Applications), which showed that the 10% cumulative diameter was 37 µm; 50% cumulative diameter (average particle diameter) was 84 µm; 90% cumulative diameter was 165 µm; and 99% cumulative diameter was 219 µm.

During the production process, problems such as precipitation of theophiline, liquid blockage, and the like were not observed in the extruder. The content of theophiline in the wax matrix granules obtained was about 100% of the theoretical value.

### Example 2

300 g of theophylline, 10 g of ethylcellulose, and 690 g of a glycerol fatty acid ester (glycerol monobehenate; melting point of about 75°C) were mixed as starting materials. Using the resulting mixture of the starting materials, wax matrix granules were produced under the same conditions as in Example 1.

The obtained wax matrix granules were spherical. The particle size distribution was measured with a laser diffraction type particle size distribution analyzer (Tohnichi Computer Applications), which showed that the 10% cumulative diameter was 43 µm; 50% cumulative diameter (average particle diameter) was 88 µm; 90% cumulative diameter was 160 µm, and 99% cumulative diameter was 204 µm.

During the production process, problems such as deposition of theophiline, liquid blockage, and the like were not observed in the extruder. The content of theophiline in the wax matrix granules obtained was 100% of the theoretical value.

### Example 3

300 g of theophylline and 700 g of hydrogenated oil (melting point of about 86°C) were mixed as starting materials. Using the resulting mixture of the starting materials, wax matrix granules were produced under the same conditions as in Example 1.

The obtained wax matrix granules were spherical. The particle size distribution was measured with a laser diffraction type particle size distribution analyzer (Tohnichi Computer Applications), which showed that the 10% cumulative diameter was 48 µm; 50% cumulative diameter (average particle diameter) was 96 µm; 90% cumulative diameter was 169 µm, and 99% cumulative diameter was 221 µm.

During the production process, problems such as deposition of theophiline, liquid blockage, and the like were not observed in the extruder. The content of theophiline in the wax matrix granules obtained was 100% of the theoretical value.

### Example 4

1350 g of cilostazol, 1710 g of diglycerol monostearate (poem J-2081, manufacture by Riken Vitamin Co., Ltd.), 990 g of pentaglycerol monostearate (sunsoft A-181E, manufactured by Taiyo Kagaku Kogyo K.K.), and 450 g of polyvinylpyrrolidone (Kollidon 25, povidone, manufactured by BASF A.G.) were mixed as starting materials. Using the resulting mixture of the starting materials, wax matrix granules were produced under the same conditions as in Example 1.

The wax matrix granules obtained were spherical. The particle size distribution was measured with a laser diffraction type particle size distribution analyzer (Tohnichi Computer Applications), which showed that the 50% cumulative diameter (average particle diameter) was about 90 µm. During the production process, problems such as deposition of cilostazol, liquid blockage, and the like were not observed in the extruder. The content of cilostazol in the wax matrix granules obtained was 100% of the theoretical value.

### Example 5

Wax matrix granules were produced using an extruder configured as shown in Fig. 2. The configuration and operation conditions of the extruder were as follows:
Extruder type: twin screw extruder (KEX-25, manufacture by Kurimoto)
Screw form: a conveyor member, a kneading member, and a mixing member are connected in series from the downstream side to the upstream side
Spray nozzle: two-fluid nozzle
Screw length: about 50 cm
Screw rotation rate: 130 rpm
Form and opening diameter of discharge port of spray nozzle:
   circular, φ 0.5 mm
   Period of time in which starting materials remained in a barrel: about two minutes
Barrel temperatures:
   140°C for a barrel jacket 1a-1
   160°C for a barrel jacket 1a-2
   165°C for barrel jacket 1a-3
   160°C for barrel jacket 1a-4
Spray air temperature and introduction rate:
   about 200°C, 25 L/min,
   Molten kneaded mixture of starting materials discharging rate per discharge port: about 50 g/minute
Atmosphere inside a granule-forming chamber:
   air, about 30°C

Specifically, 240 g of cilostazol having an average particle diameter of about 20 µm, 348 g of diglycerol monostearate (poem J-2081, manufactured by Riken Vitamin Co., Ltd.), and 12 g of triglyceryl half-ester of behenic acid (TR-HB, manufactured by Riken Vitamin Co., Ltd.) were mixed. While the resulting mixture of the starting materials was supplied to a supply port of the above-mentioned extruder at about 50 g/min, wax matrix granules were produced by an extruder configured as described above and having the above-described conditions, and the wax matrix granules produced were then collected from a wax matrix granule collector of the granule-forming chamber.

The obtained wax matrix granules had high aggregability, but the flowability was increased by adding and mixing 14.8 g of talc. The wax matrix granules thus obtained passed through a sieve with a mesh opening of 355 µm or less. Subsequently, the sized wax matrix granules were heated at 50°C for 16 hours.

The obtained wax matrix granules were observed under a microscope, and found that cilostazol crystals whose particle diameter is 10 µm or less formed. The obtained wax matrix granules were spherical. The particle size distribution was measured with a laser diffraction type particle size distribution analyzer (Tohnichi Computer Applications), which showed that the average particle diameter (50% cumulative diameter) was 92 µm. During the production process, problems such as precipitation of cilostazol, liquid blockage, and the like were not observed in the extruder.

### Example 6

240 g of cilostazol having an average particle diameter of about 20 µm, 336 g of diglycerol monostearate (poem J-2081, manufactured by Riken Vitamin Co., Ltd.), and 24 g of triglyceryl half-ester of behenic acid (TR-HB, manufactured by Riken Vitamin Co. , Ltd.) were mixed as starting materials. Using the resulting mixture of the starting materials, wax matrix granules were produced under the same conditions as in Example 1.

The obtained wax matrix granules were observed under a microscope, and found that cilostazol crystals whose particle diameter is 10 µm or less formed. The obtained wax matrix granules were spherical. The particle size distribution was measured with a laser diffraction type particle size distribution analyzer (Tohnichi Computer Applications), which showed that the average particle diameter (50% cumulative diameter) was 93 µm. During the production process, problems such as precipitation of cilostazol, liquid blockage, and the like were not observed in the extruder.

### Example 7

240 g of cilostazol having an average particle diameter of about 20 µm, 324 g of diglycerol monostearate (poem J-2081, manufactured by Riken Vitamin Co., Ltd.), and 36 g of triglyceryl half-ester of behenic acid (TR-HB, manufactured by Riken Vitamin Co., Ltd.) were mixed as starting materials. Using the resulting mixture of the starting materials, wax matrix granules were produced under the same conditions as in Example 5.

The obtained wax matrix granules were observed under a microscope, and found that cilostazol crystals whose particle diameter is 10 µm or less formed. The obtained wax matrix granules were spherical. The particle size distribution was measured with a laser diffraction type particle size distribution analyzer (Tohnichi Computer Applications), which showed that the average particle diameter (50% cumulative diameter) was 91 µm. During the production process, problems such as precipitation of cilostazol, liquid blockage, and the like were not observed in the extruder.

### Example 8

240 g of cilostazol having an average particle diameter of about 20 µm, 234 g of diglycerol monostearate (poem J-2081, manufactured by Riken Vitamin Co., Ltd.), 24 g of triglyceryl half-ester of behenic acid (TR-HB, manufactured by Riken Vitamin Co., Ltd.), and 102 g of glycerol behenate (poem B-100, manufactured by Riken Vitamin Co., Ltd.) were mixed as starting materials. Using the resulting mixture of the starting materials, wax matrix granules were produced under the same conditions as in Example 5.

The obtained wax matrix granules were observed under a microscope, and found that cilostazol crystals whose particle diameter is 10 µm or less formed. The obtained wax matrix granules were spherical. The particle size distribution was measured with a laser diffraction type particle size distribution analyzer (Tohnichi Computer Applications), which showed that the average particle diameter (50% cumulative diameter) was 79 µm. During the production process, problems such as precipitation of cilostazol, liquid blockage, and the like were not observed in the extruder.

### Example 9

240 g of cilostazol having an average particle diameter of about 20 µm, 222 g of diglycerol monostearate (poem J-2081V, manufactured by Riken Vitamin Co., Ltd.), 24 g of triglyceryl half-ester of behenic acid (TR-HB, manufactured by Riken Vitamin Co., Ltd.), 96 g of glycerol behenate (poem B-100, manufactured by Riken Vitamin Co., Ltd.), and 18 g of hydroxypropylmethylcellulose (TC-5E, manufactured by Shinetsu Kagaku Co., Ltd.) were mixed as starting materials. Using the resulting mixture of the starting materials, wax matrix granules were produced under the same conditions as in Example 5 except that some conditions were changed as follows:
Barrel temperatures:
   120°C for a barrel jacket 1a-1
   185°C for a barrel jacket 1a-2
   185°C for barrel jacket 1a-3
   185°C for barrel jacket 1a-4
Spray air temperature and introduction rate:
   about 200°C, 50 L/min,

Molten kneaded mixture of starting materials discharging rate per discharge port: 118 g/minute.

12.6 g of talc was added to and mixed with 314 g of the obtained wax matrix granules, and the result was heated at 50°C for 16 hours, followed by sizing using a sieve with a mesh opening of 350 µm, giving sized wax matrix granules.

The wax matrix granules were observed under a microscope, and found that cilostazol crystals whose particle diameter is larger than 10 µm were not formed. The obtained wax matrix granules were spherical. The particle size distribution was measured with a laser diffraction type particle size distribution analyzer (Tohnichi Computer Applications), which showed that the average particle diameter (50% cumulative diameter) was about 77 µm. During the production process, problems such as precipitation of cilostazol, liquid blockage, and the like were not observed in the extruder.

### Example 10

240 g of cilostazol having an average particle diameter of about 20 µm, 210 g of diglycerol monostearate (poem J-2081V, manufactured by Riken Vitamin Co., Ltd.), 24 g of triglyceryl half-ester of behenic acid (TR-HB, manufactured by Riken Vitamin Co. , Ltd.), 90 g of glycerol behenate (poem B-100, manufactured by Riken Vitamin Co., Ltd.), and 36 g of hydroxypropylmethylcellulose (TC-5E, manufactured by Shinetsu Kagaku Co., Ltd.) were mixed as starting materials. Using the resulting mixture of the starting materials, wax matrix granules were produced under the same conditions as in Example 5 except that some conditions were changed as follows:
Barrel temperatures:
   120°C for a barrel jacket 1a-1
   185°C for a barrel jacket 1a-2
   185°C for barrel jacket 1a-3
   185°C for barrel jacket 1a-4
Spray air temperature and introduction rate:
   about 200°C, 40 L/min,

Molten kneaded mixture of starting materials discharging rate per discharge port: about 175 g/minute.

14.1 g of talc was added to and mixed with 353 g of the obtained wax matrix granules, and the result was heated at 50°C for 16 hours, followed by sizing using a sieve with a mesh opening of 350 µm, giving sized wax matrix granules.

The wax matrix granules were observed under a microscope, and found that cilostazol crystals whose particle diameter is larger than 10 µm were not formed. The obtained wax matrix granules were spherical. The particle size distribution was measured with a laser diffraction type particle size distribution analyzer (Tohnichi Computer Applications), which showed that the average particle diameter (50% cumulative diameter) was about 104 µm. During the production process, problems such as precipitation of cilostazol, liquid blockage, and the like were not observed in the extruder.

### Example 11

240 g of cilostazol having an average particle diameter of about 20 µm, 198 g of diglycerol monostearate (poem J-2081V, manufactured by Riken Vitamin Co., Ltd.), 24 g of triglyceryl half-ester of behenic acid (TR-HB, manufactured by Riken Vitamin Co. , Ltd.), 84 g of glycerol behenate (poem B-100, manufactured by Riken Vitamin Co., Ltd.), and 54 g of hydroxypropylmethylcellulose (TC-5E, manufactured by Shinetsu Kagaku Co., Ltd.) were mixed as starting materials. Using the resulting mixture of the starting materials, wax matrix granules were produced under the same conditions as in Example 5 except that some conditions were changed as follows:
Barrel temperatures:
   120°C for a barrel jacket 1a-1
   185°C for a barrel jacket 1a-2
   185°C for barrel jacket 1a-3
   185°C for barrel jacket 1a-4
Spray air temperature and introduction rate:
   about 200°C, 50 L/min,

Molten kneaded mixture of starting materials discharging rate per discharge port: about 120 g/minute.

10.7 g of talc was added to and mixed with 267 g of the obtained wax matrix granules, and the result was heated at 50°C for 16 hours, followed by sizing using a sieve with a mesh opening of 350 µm, giving sized wax matrix granules.

The wax matrix granules were observed under a microscope, and found that cilostazol crystals whose particle diameter is larger than 10 µm were not formed. The obtained wax matrix granules were spherical. The particle size distribution was measured with a laser diffraction type particle size distribution analyzer (Tohnichi Computer Applications), which showed that the average particle diameter (50% cumulative diameter) was about 93 µm. During the production process, problems such as precipitation of cilostazol, liquid blockage, and the like were not observed in the extruder.

### Example 12

240 g of cilostazol having an average particle diameter of about 20 µm, 222 g of diglycerol monostearate (poem J-2081V, manufactured by Riken Vitamin Co., Ltd.), 24 g of triglyceryl half-ester of behenic acid (TR-HB, manufactured by Riken Vitamin Co., Ltd.), and 60 g of hydroxypropylmethylcellulose (TC-5E, manufactured by Shinetsu Kagaku Co., Ltd.) were mixed as starting materials. Using the resulting mixture of the starting materials, wax matrix granules were produced under the same conditions as in Example 5 except that some conditions were changed as follows:
Barrel temperatures:
   130°C for a barrel jacket 1a-1
   165°C for a barrel jacket 1a-2
   175°C for barrel jacket 1a-3
   170°C for barrel jacket 1a-4
Spray air temperature and introduction rate:
   about 200°C, 50 L/min,

Molten kneaded mixture of starting materials discharging rate per discharge port: about 140 g/minute.

12.8 g of talc was added to and mixed with 320 g of the obtained wax matrix granules, and the result was heated at 50°C for 16 hours, followed by sizing using a sieve with a mesh opening of 350 µm, giving sized wax matrix granules.

The wax matrix granules were observed under a microscope, and found that cilostazol crystals whose particle diameter is larger than 10 µm were not formed. The obtained wax matrix granules were spherical. The particle size distribution was measured with a laser diffraction type particle size distribution analyzer (Tohnichi Computer Applications), which showed that the average particle diameter (50% cumulative diameter) was about 98 µm. During the production process, problems such as precipitation of cilostazol, liquid blockage, and the like were not observed in the extruder.

### Example 13

240 g of cilostazol having an average particle diameter of about 20 µm, 228 g of diglycerol monostearate (poem J-2081V, manufactured by Riken Vitamin Co., Ltd.), 48 g of triglyceryl half-ester of behenic acid (TR-HB, manufactured by Riken Vitamin Co., Ltd.), 60 g of glycerol behenate (poem B-100, manufactured by Riken Vitamin Co., Ltd.), and 24 g of carboxyvinyl polymer (Carbopol 974P) were mixed as starting materials. Using the resulting mixture of the starting materials, wax matrix granules were produced under the same conditions as in Example 5 except that some conditions were changed as follows:
Barrel temperatures:
   120°C for a barrel jacket 1a-1
   185°C for a barrel jacket 1a-2
   185°C for barrel jacket 1a-3
   185°C for barrel jacket 1a-4
Spray air temperature and introduction rate:
   about 200°C, 45 L/min,

Molten kneaded mixture of starting materials discharging rate per discharge port: about 128 g/minute.

15.4 g of talc was added to and mixed with 384 g of the obtained wax matrix granules, and the mixture was heated at 50°C for 16 hours, followed by sizing using a sieve with a mesh opening of 350 µm, giving sized wax matrix granules.

The wax matrix granules were observed under a microscope, and found that cilostazol crystals whose particle diameter is larger than 10 µm were not formed. The obtained wax matrix granules were spherical. The particle size distribution was measured with a laser diffraction type particle size distribution analyzer (Tohnichi Computer Applications), which showed that the average particle diameter (50% cumulative diameter) was about 135 µm. During the production process, problems such as precipitation of cilostazol, liquid blockage, and the like were not observed in the extruder.

### Example 14

1.0 g of light anhydrous silicic acid (Adsolider 101/YKF) was further added to 260g of the wax matrix granules obtained in Example 13. 261 mg of the obtained mixture was placed in a hard capsule, giving a capsule agent.

### Comparative Example 1

1350 g of cilostazol, 1710 g of diglycerol monostearate (poem J-2081, manufactured by Riken Vitamin Co. , Ltd.), 990 g of pentaglycerol monostearate (sunsoft A-181E, manufactured by Taiyo Kagaku Kogyo K.K.), and 450 g of polyvinylpyrrolidone (Kollidon25, povidone, manufactured by BASF A.G.) were put in a stirring tank with a closed jacket. The mixture was kneaded while heating at 150°C, preparing a transparent molten kneaded liquid. This molten kneaded liquid was fed by pressure to a rotation-disk-type spray air cooler (diameter of 2.5 m) from the tank. The piping from the tank to the disk (about 60 cm length) was heated at about 150°C with a ribbon heater. Cilostazol precipitated out in the middle of the piping, resulting in a pipe blockage, which precluded spraying of the resulting mixture. This result showed that wax matrix granules were not produced according to the method of Comparative Example 1 even if the same starting materials as in Example 4 were used.

### Comparative example 2

A molten liquid was prepared under the same conditions as in Comparative Example 1 using 1000 g of cilostazol, 1800 g of diglycerol monostearate (poem J-2081, manufactured by Riken Vitamin Co., Ltd.), 400 g of polyvinylpyrrolidone (Kollidon25, povidone, manufactured by BASF A.G.), 800 g of glycerol monostearate citrate (sunsoft No. 621G, manufactured by Taiyo Kagaku Kogyo K.K.). The resulting molten liquid was supplied to a spray cooler, and then sprayed and cooled with a rotation disk, preparing granules. As a result, only a slight amount of wax matrix granules were obtained. However, cilostazol crystals precipitated out on the liquid-contacting surface in the tank, on the shaft, and inside the piping. The content of cilostazol in the wax matrix granules obtained was 45% of the theoretical value.

### Test Example 1 Dissolution Test

The wax matrix granules obtained in Examples 5 to 8 were evaluated for their cilostazol release property. More specifically, a dissolution test was performed on the wax matrix granules (Examples 5 to 8) in an amount equivalent to a cilostazol content of 15 mg, using 900 mL of a 1 wt.% aqueous polysorbate 80 solution as an eluate at a paddle rotation of 75 rpm according to the paddle method (method 2) of the dissolution test described in the Japanese Pharmacopoeia 14th Edition to determine the amount of cilostazol dissolved in the eluate over time (i.e., measured at the two wavelengths of 257 nm and 325 nm) and to calculate the percentage of cilostazol eluted from the wax matrix granules (dissolution rat) (%).

Fig. 4 shows the results. The results confirm that all of the wax matrix granules obtained in Examples 5 to 8 exhibit an ideal dissolution behavior for sustained-release preparations.

### Test Example 2 Pharmacokinetic Evaluation

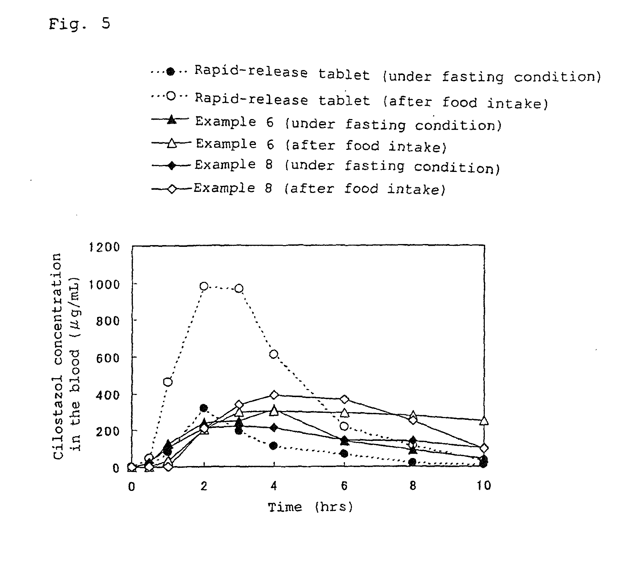

Capsule preparations were prepared by placing the wax matrix granules of Example 6 or 8 in an amount equivalent to a cilostazol content of 100 mg into a gelatin capsule. One gelatin capsule preparation thus prepared was orally administered to each of three beagles under fasting conditions or after food intake, and their blood samples were collected over time to determine the blood cilostazol concentration. Likewise, commercially available Pletal tablets (rapid-release tablets) (in an amount equivalent to a cilostazol content of 100 mg and containing crystalline cellulose, corn starch, carmellose calcium, hydroxypropylmethylcellulose, and magnesium stearate) were orally administered, and blood samples were collected over time to determine the blood cilostazol concentration. Fig. 5 shows a comparison of the blood cilostazol concentration change, and Table 1 shows the pharmacokinetic parameters calculated.

When administering the rapid-release tablets, great differences were observed between administration while fasting and administration after food intake in Cₘₐₓ and AUC; and the results were influenced by food intake. In contrast, when administering the wax matrix granules of Example 6 or 8, only small differences were observed between administration while fasting and administration after food intake in Cₘₐₓ and AUC; and it was confirmed that the results were hardly influenced by food intake.

**[Table 1]**

| | | AUCt (ng.hr/mL) | AUC∞ (ng.hr/mL) | Cmax (ng/mL) | Tmax (hr) | MRTt (hr) |
|---|---|---|---|---|---|---|
| | Value while fasting | 1576 | 1748 | 346 | 5.3 | 4.46 |
| Example 2 | Value after food intake | 2373 | 4654 | 402 | 6.3 | 5.89 |
| | Value after food intake / value while fasting ratio (%) | 151% | 266% | 116% | 119% | 132% |
| | Value while fastening | 916 | 947 | 322 | 2.3 | 3.44 |
| Rapid release tablet | Value after food intake | 3933 | 4010 | 1198 | 2.3 | 3.46 |
| | Value after food intake / value while fasting ratio (%) | 429% | 423% | 372% | 100% | 101% |

| | | | | | | |
|---|---|---|---|---|---|---|
| AUCt: Area under the blood cilostazol concentration-time curve from time zero to the last sampling time (trapezoidal rule) AUC∞: Area under the blood cilostazol concentration-time curve to infinite time after administration Cₘₐₓ: Maximum blood cilostazol concentration Tₘₐₓ: Time required to reach maximum blood cilostazol concentration MRTₜ: Mean residence time | | | | | | |

### Test Example 3 Pharmacokinetic Evaluation

Capsule preparations were prepared by placing wax matrix granules of Example 10 or 13 in an amount equivalent to a cilostazol content of 100 mg into a gelatin capsule. One capsule preparation thus prepared was orally administered to each of three beagles after food intake, and their blood samples were collected over time to determine the blood cilostazol concentration. Table 2 shows the pharmacokinetic parameters calculated from the blood cilostazol concentrations.

The results confirm that the wax matrix granules of Examples 10 and 13 exhibit an especially good dissolution behavior as sustained-release preparations. The results clearly show that when a preparation comprises wax matrix granules containing hydroxypropylmethylcellulose in addition to cilostazol crystals and a glycerine fatty acid ester and/or a polyglycerine fatty acid ester, the preparation can more effectively exhibit the dissolution behavior required of sustained-release preparations.

**[Table 2]**

| | AUCt (ng.hr/mL) | AUC∞ (ng.hr/mL) | Cmax (ng/mL) | Tmax (hr) | MRTt (hr) |
|---|---|---|---|---|---|
| Example 13 | 1605 | 2268 | 457 | 3.0 | 4.20 |
| Example 10 | 3296 | 3482 | 758 | 3.5 | 4.45 |

| | | | | | |
|---|---|---|---|---|---|
| AUCt: Area under the blood cilostazol concentration-time curve from time zero to the last sampling time (trapezoidal rule) AUC∞: Area under the blood cilostazol concentration-time curve to infinite time after administration Cₘₐₓ: Maximum blood cilostazol concentration Tₘₐₓ: Time required to reach maximum blood cilostazol concentration MRTₜ: Mean residence time | | | | | |

### BRIEF DESCRIPTION OF THE DRAWINGS

Fig. 1 is a partially cut away side view showing one example of an extruder for producing drug-containing wax matrix granules.
Fig. 2 is a side view showing one example of an extruder having a granule-forming chamber for producing drug-containing wax matrix granules.
Fig. 3 is a micrograph of the wax matrix granules obtained in Example 1 observed under a microscope. The bar scale shown in the micrograph represents 200 µm.
Fig. 4 is a graph showing dissolution characteristics of wax matrix granules (Examples 5 to 8) determined in Test Example 1.
Fig. 5 is a graph showing changes in the average blood cilostazol concentration over time after administration of the wax matrix granules (Examples 6 and 8) and those after administration of the rapid-release tablets determined in Test Example 2.

### DESCRIPTION OF REFERENCE NUMERALS

1. Barrel
2. Supply port
3. Outlet die
4. Screw
5. Spray nozzle
6. Granule-forming chamber
7. Exhaust member
10. Wax matrix granules discharged from a discharge port 5b of the spray nozzle

## Claims

1. A sustained-release preparation containing granules comprising:
(A) cilostazol crystals; and
(B) glycerol fatty acid ester and/or polyglycerol fatty acid ester;
and an average particle diameter of the granules ranges from 40 to 200 µm.

2. A sustained-release preparation according to claim 1, wherein an average particle diameter of the (A) cilostazol crystals is 10 µm or less.

3. A sustained-release preparation according to claim 1, comprising (A) cilostazol crystals having a particle diameter of 10 µm or less.

4. A sustained-release preparation according to any one of claims 1 to 3, wherein (A) the cilostazol crystals are present in a proportion of 5 to 60% by weight and (B) the glycerol fatty acid ester and/or polyglycerol fatty acid ester is/are present in a proportion of 30 to 95% by weight based on a total amount of the granules in the sustained-release preparation.

5. A sustained-release preparation according to any one of claims 1 to 3, further comprising a water-soluble cellulose derivative.

6. A sustained-release preparation according to claim 5, wherein the water-soluble cellulose derivative is hydroxypropylmethylcellulose.

7. A sustained-release preparation according to claim 5, comprising 1 to 15% by weight of the water-soluble cellulose derivative based on a total amount of the wax matrix granules contained in the preparation.

8. A sustained-release preparation according to claim 6, comprising 1 to 15% by weight of hydroxypropylmethylcellulose based on a total amount of the wax matrix granules contained in the preparation.

9. A sustained-release preparation according to any one of claims 1 to 3, wherein inert particles are adhered to a surface of the granules.

10. A sustained-release preparation according to claim 9, wherein the inert particle is at least one member selected from the group consisting of talc, light anhydrous silicic acid, titanium oxides, and cellulose-based polymers.

11. A sustained-release preparation according to any one of claims 1 to 3, wherein the ingredient (B) is at least one member selected from the group consisting of glycerol stearate, polyglycerol stearate, glycerol behenate, and polyglycerol behenate.

12. A sustained-release preparation according to any one of claims 1 to 3, wherein the ingredient (B) is at least one member selected from the group consisting of glycerol behenate, diglycerol stearate, and triglyceryl half-ester of behenic acid.

13. A method for producing a sustained-release preparation of any one of claims 1 to 3, which comprises the preparation of granules by solidifying a molten mixture comprising (A) cilostazol and (B) glycerol fatty acid ester and/or polyglycerol fatty acid ester.

14. The method according to claim 13, which comprises the steps of:
(i) supplying (A) cilostazol and (B) glycerol fatty acid ester and/or polyglycerol fatty acid ester to an extruder in which the temperature of a barrel and the temperature of a die are adjusted to be higher than the melting point of the ingredient (B); and
(ii) while melting and kneading the ingredients (A) and (B) in the extruder to give a molten kneaded mixture of the ingredients (A) and (B), spraying the molten kneaded mixture of the ingredients (A) and (B) into an atmosphere having a temperature lower than the melting point of the ingredient (B) from a spray nozzle directly mounted onto a die provided at a top end of the barrel of the extruder, thereby forming the mixture into granules.

15. The method according to claim 14, wherein (C) a water-soluble cellulose derivative is supplied in addition to the ingredients (A) and (B) in the step (i).

16. The method according to claim 15 or 16, further comprising the step (iii) of heating the granules prepared in the step (ii) at a temperature of 40 to 55°C.

17. The method according to claim 16, wherein, in the step (iii), inert particles are adhered to a surface of the granules obtained in the step (ii) before heating the granules at a temperature of 40 to 55°C.
